# EUROPEAN PATENT SPECIFICATION

(11) **EP 0 581 502 B1**
(45) Date of publication and mention of the grant of the patent: **21.10.1998**
(21) Application number: 93305609.5
(22) Date of filing: 16.07.1993
(51) Int. Cl.: C07D 477/00, C07F 9/568, C07D 417/12, C07D 403/12, C07D 207/34, C07D 417/14, C07D 403/14, A61K 31/40

(54) **Antibiotic carbapenem compounds**
Antibiotische carbapenemverbindungen
Dérivés de carbapénème antibiotiques

(30) Priority: 21.07.1992 EP 92402103
(43) Date of publication of application: 02.02.1994
(73) Proprietor: ZENECA LIMITED, London W1Y 6LN (GB); ZENECA Pharma S.A., 95022 Cergy Pontoise Cédex (FR)
(72) Inventor: Jung, Frederic Henri, F-51064 Reims Cédex (FR); Lohmann, Jean Jacques, F-51064 Reims Cédex (FR)
(74) Representative: Denerley, Paul Millington, Dr.

(56) References cited:
- EP-A- 0 126 587
- EP-A- 0 443 883
- EP-A- 0 518 558
- WO-A-92/17481

## Description

The present invention relates to carbapenems and in particular to such compounds containing a carboxy substituted 5-membered heterocyclic ring. This invention further relates to processes for their preparation, to intermediates in their preparation, to their use as therapeutic agents and to pharmaceutical compositions containing them. The compounds of this invention are antibiotics and can be used in the treatment of any disease that is conventionally treated with antibiotics for example in the treatment of bacterial infection in mammals including humans.

Carbapenems were first isolated from fermentation media in 1974 and were found to have broad spectrum antibacterial activity. Since this discovery substantial investigations have been made into new carbapenem derivatives and many hundreds of patents and scientific papers have been published.

The first carbapenem, and so far the only, to be commercially marketed is imipenem (N-formididoyl thienamycin). This compound has a broad spectrum of antibacterial activity.

European patent application, publication no. 126587 discloses carbapenem compounds containing a carbamoyl pyrrrolidinylthio 2-position substitutuent in which the carbamoyl group may be substituted by aralkyl or pyridyl. European patent application no. 443 883 discloses carbapenem compounds which contain a 2-position pyrrolidinylthio group which can be substituted by a range of groups containing a quaternary nitrogen. Heteroaralkylcarbamoyl, wherein the heteroaryl ring contains a quaternised nitrogen, is an example of one group of substituents this application discloses for the pyrrolidinylthio group.

The present invention provides compounds with a broad spectrum of antibacterial activity including both Gram positive and negative, aerobic and anaerobic bacteria. They exhibit good stability to beta-lactamases. In addition representative compounds of this invention exhibit favourable pharmacokinetics.

The carbapenem derivatives referred to herein are named in accordance with the generally accepted semi-systematic nomenclaure: 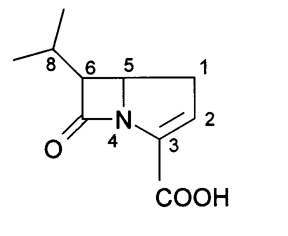 Accordingly the present invention provides a compound of the formula (I) 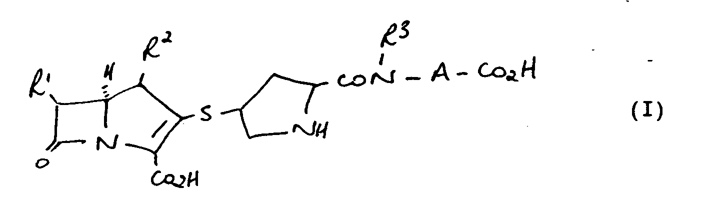 wherein:
R¹ is 1-hydroxyethyl, 1-fluoroethyl or hydroxymethyl;
R² is hydrogen or C₁₋₄alkyl;
R³ is hydrogen or C₁₋₄alkyl;
A is a 5-membered heteroaryl ring containing one nitrogen atom and up to two additional heteroatoms selected from nitrogen, oxygen and sulphur; and is bonded to the nitrogen of the linking carbamoyl group by a carbon atom in the ring, is substituted with the carboxy group on a carbon atom in the ring, and is optionally further substituted on a carbon atom in the ring, by halo, cyano, C₁₋₄alkyl, nitro, hydroxy, carboxy, C₁₋₄alkoxy, trifluoromethyl, C₁₋₄alkoxycarbonyl, amino, C₁₋₄alkylamino, di-C₁₋₄alkylamino, C₁₋₄alkylS(O)ₙ- (wherein n is 0-2), C₁₋₄alkanoylamino, C₁₋₄alkanoyl(N-C₁₋₄alkyl)amino, carbamoyl, C₁₋₄alkylcarbamoyl or di-C₁₋₄alkylcarbamoyl; and
   in any ring -NH-, H is optionally replaced by C₁₋₄alkyl; or a pharmaceutically acceptable salt or in vivo hydrolysable ester thereof.

The term alkyl includes all straight and branched chain structures, for example, C₁₋₄alkyl includes n-butyl and 2-methylpropyl.

The term heteroaryl means an aromatic ring containing at least one heteroatom in the ring.

Preferably R¹ is 1-hydroxyethyl.

R² is hydrogen or C₁₋₄alkyl for example methyl, ethyl, n-propyl, 1-methylethyl and n-butyl.

Preferably R² is hydrogen or methyl and in particular R² is methyl.

R³ is hydrogen or C₁₋₄alkyl for example methyl, ethyl, n-propyl, 1-methylethyl and n-butyl.

Preferably R³ is hydrogen or methyl.

Most preferably R³ is hydrogen.

Preferably A is thiazole, 1,2,4-thiadiazole, 1,3,4-thiadiazole, oxazole, pyrrole, pyrazole, oxadiazole, imidazole, isoxazole, 1,2,4-thiadiazole or isothiazole. Most preferably A is thiazole, 1,3,4-thiadiazole, pyrrole or imidazole.

Preferably any nitrogen atoms in the heteroaryl ring are unsubstituted.

Suitable substituents for A include, for example:-
- for halo:: fluoro, chloro and bromo;
- for C₁₋₄alkyl:: methyl, ethyl propyl, 1-methylethyl, butyl and 2-methylpropyl;
- for C₁₋₄alkoxy:: methoxy, ethoxy, propoxy, 1-methylethoxy, butoxy and 2-methylpropoxy;
- for C₁₋₄alkylcarbomoyl:: methylcarbamoyl, ethylcarbamoyl and propylcarbamoyl;
- for di-C₁₋₄alkylcarbamoyl:: dimethylcarbamoyl and diethylcarbamoyl;
- for C₁₋₄alkylamino:: methylamino, ethylamino and propylamino;
- for di-C₁₋₄alkylamino:: dimethylamino, diethylamino and methylethylamino;
- for C₁₋₄alkylS(O)ₙ-:: methylthio, methylsulphinyl and methylsulphonyl;
- for C₁₋₄alkanoylamino:: acetamido and propionamido;
- for C₁₋₄alkanoyl(N-C₁₋₄alkyl)amino:: N-methylacetamido and N-ethylacetamido;
- for C₁₋₄alkoxycarbonyl:: methoxycarbonyl and ethoxycarbonyl.

Preferably, when A is optionally substituted, the optional substituents are selected from halo, cyano, C₁₋₄alkyl, nitro, carboxy, hydroxy, C₁₋₄alkoxy, carbamoyl, amino and trifluoromethyl.

The present invention covers all epimeric, diastereoisomeric and tautomeric forms of the compounds of the formula (I) wherein the absolute stereochemistry at the 5-position is as illustrated in formula (I). When a bond is represented as a wedge, this indicates that in three dimensions the bond would be coming forward out of the paper and when a bond is represented as hatched, this indicates that in three dimensions the bond would be going back into the paper. The compounds of the formula (I) have a number of other centres of optical activity, namely: within the group R¹ (when R¹ is 1-hydroxyethyl or 1-fluoroethyl); at the 6-position; at the 1-position (when R² is C₁₋₄alkyl); and at the 2' and 4' positions in the pyrrolidine ring:

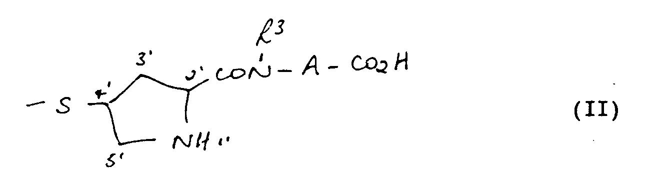

Preferred compounds are those in which the beta-lactam protons are in trans configuration with respect to one another. When R¹ is 1-hydroxyethyl or 1-fluoroethyl it is preferred that the 8-substituent has the R-configuration. Thus a preferred class of compounds is that of the formula (III): 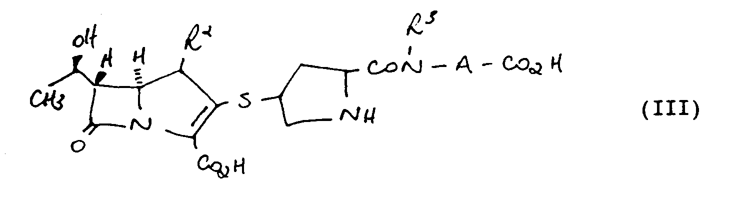 and pharmaceutically acceptable salts and in vivo hydrolysable esters thereof, wherein R², R³ and optional substituents on the heteroaryl ring are as hereinbefore defined.

When R² is C₁₋₄alkyl for example methyl it is preferred that the compound is in the form of the 1R configuration.

Preferred compounds are those in which the pyrrolidine ring has the following absolute stereochemistry at the 2'- and 4'-positions:

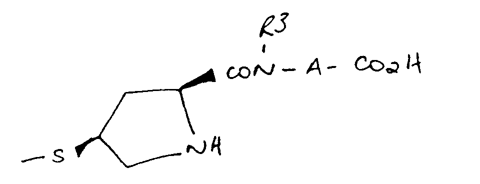

A suitable class of compounds of the present invention is that of the formula (IV): 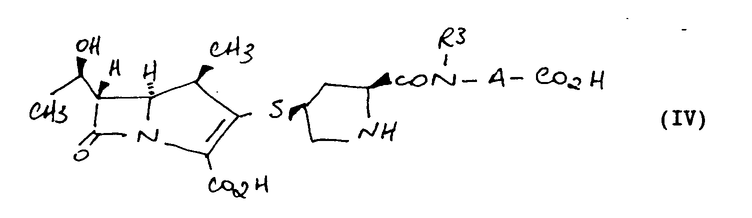 and pharmaceutically acceptable salts and in vivo hydrolysable esters thereof;
wherein A, R³ and optional substituents on A are as defined hereinbefore in formula (I).

In another aspect a suitable class of compounds are the compounds of the formula (IV) wherein R³ is hydrogen, methyl or ethyl; and A and optional substituents on A are as defined hereinabove in formula (I).

In yet another aspect a suitable class of compounds is that of the compounds of the formula (IV) wherein A is optionally further substituted by one or two substituents selected from methyl, ethyl, hydroxy, carboxy, cyano, fluoro, chloro, bromo, carbamoyl, nitro, methoxy, ethoxy and propoxy; and A and R³ are as defined hereinbefore in formula (I).

A particular class of compounds of the present invention is that of the formula (IV) wherein:
R³ is hydrogen or methyl;
A is as hereinabove defined;
and A is optionally further substituted by one substituent selected from methyl, ethyl, hydroxy, carboxy, cyano, chloro, bromo, nitro, methoxy and ethoxy.

A preferred class of compounds of the present invention is that of the formula (IV) wherein:
R³ is hydrogen;
A is as hereinabove defined;
and A is optionally further substituted by one substituent selected from methyl, hydroxy, chloro and carboxy.

A more preferred class of compounds of the present invention is that of the formula (IV) wherein:
R³ is hydrogen;
A is as hereinabove defined;
and A is not further substituted.

An even more preferred class of compounds of the present invention is that of the formula (IV) wherein:
R³ is hydrogen;
A is thiazole, 1,3,4-thiadiazole, pyrrole or imidazole;
and A is not further substituted.

Particular compounds of the present invention are, for example, the following compounds of the formula (IV):
(1R,5S,6S,8R,2'S,4'S)-2-(2-(5-carboxythiazol-2-ylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(2-carboxy-1H-imidazol-4-ylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(2-carboxypyrrol-4-ylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(4-carboxythiazol-2-ylcarbamoyl)pyrrolidin-4-ylthio)-6-(1-hydroxyethyl)-1-methylcarbapenem-3-carboxylic acid; and
(1R,5S,6S,8R,2'S,4'S)-2-(2-(5-carboxy-1,3,4-thiadiazol-2-ylcarbamoyl)pyrrolidin-4-ylthio)-6-(1-hydroxyethyl)-1-methylcarbapenem-3-carboxylic acid;
and pharmaceutically acceptable salts and in vivo hydrolysable esters thereof.

Suitable pharmaceutically acceptable salts include acid addition salts such as hydrochloride, hydrobromide, citrate, maleate and salts formed with phosphoric and sulfuric acid. In another aspect suitable salts are base salts such as an alkali metal salt for example sodium or potassium, an alkaline earth metal salt for example calcium or magnesium, an organic amine salt for example triethylamine, morpholine, N-methylpiperidine, N-ethylpiperidine, procaine, dibenzylamine, N,N-dibenzylethylamine or aminoacids, for example, lysine.

Preferred pharmaceutically acceptable salts are sodium and potassium salts. However, to facilitate isolation of the salt during preparation, salts which are less soluble in the chosen solvent may be preferred, whether pharmaceutically acceptable or not.

For the avoidance of doubt there may be one, two, three or four salt-forming cations depending on the number of carboxylic acid functions and valency of said cations.

In vivo hydrolysable esters are those pharmaceutically acceptable esters that hydrolyse in the human body to produce the parent hydroxy or carboxy compound. Such esters can be identified by administering, eg. intravenously to a test animal, the compound under test and subsequently examining the test animal's body fluids. Suitable in vivo hydrolysable esters for hydroxy include acetoxy, propionyloxy, pivaloyloxy, C₁₋₄alkoxycarbonyloxy for example ethoxycarbonyloxy, phenylacetoxy and phthalidyl. Suitable in vivo hydrolysable esters for carboxy include C₁₋₆alkoxymethyl esters for example methoxymethyl; C₁₋₆alkanoyloxymethyl esters for example pivaloyloxymethyl; C₃₋₈ cycloalkoxycarbonyloxyC₁₋₆alkyl, for example 1-cyclohexyloxycarbonyloxyethyl; 1,3-dioxolen-2-onylmethyl esters for example 5-methyl-1,3-dioxolen-2-onylmethyl; phthalidyl esters and C₁₋₆alkoxycarbonyloxyethyl esters for example 1-ethoxycarbonyloxyethyl and may be formed at any carboxy group in the compounds of this invention.

In order to use a compound of the formula (I) or a pharmaceutically acceptable salt or in vivo hydrolysable ester thereof for the therapeutic treatment of mammals including humans, in particular in treating infection, it is normally formulated in accordance with standard pharmaceutical practice as a pharmaceutical composition.

Therefore in another aspect the present invention provides a pharmaceutical composition which comprises a compound of the formula (I) or a pharmaceutically acceptable salt or in vivo hydrolysable ester thereof and a pharmaceutically acceptable carrier.

The pharmaceutical compositions of this invention may be administered in standard manner for the disease condition that it is desired to treat, for example by oral, rectal or parenteral administration. For these purposes the compounds of this invention may be formulated by means known in the art into the form of, for example, tablets, capsules, aqueous or oily solutions or suspensions, emulsions, dispersible powders, suppositories and sterile injectable aqueous or oily solutions or suspensions.

The compounds of the present invention may be formulated as dry powder filled vials, which may contain the compound of the present invention alone or as a dry blended mixture. For example an acidic compound of the present invention may be dry blended with an alkali metal carbonate or bicarbonate. Freeze dried formulations of compounds of the present invention, alone or as a mixture with standard excipients, are possible. Standard excipients include structure formers, cryoprotectants and pH modifiers, such as, mannitol, sorbitol, lactose, glucose, sodium chloride, dextran, sucrose, maltose, gelatin, bovine serum albumin (BSA), glycine, mannose, ribose, polyvinylpyrrolidine (PVP), cellulose derivatives, glutamine, inositol, potassium glutamate, erythritol, serine and other amino acids and buffer agents e.g. disodium hydrogen phosphate and potassium citrate.

In addition to the compounds of the present invention the pharmaceutical composition of this invention may also contain, or be co-administered with, one or more known drugs selected from other clinically useful antibacterial agents (for example other beta-lactams or aminoglycosides), inhibitors of beta-lactamase (for example clavulanic acid), renal tubular blocking agents (e.g. probenecid) and inhibitors of metabolising enzymes (for example inhibitors of dehydropeptidases, for example Z-2-acylamino-3-substituted propenoates such as cilastatin) and N-acylated amino acids such as betamipron (also see EP-A-178911).

A suitable pharmaceutical composition of this invention is one suitable for oral administration in unit dosage form, for example a tablet or capsule which contains between 100mg and 1g of the compound of this invention.

A preferred pharmaceutical composition of the invention is one suitable for intravenous, subcutaneous or intramuscular injection, for example a sterile injectable composition containing between 1 and 50% w/w of the compound of this invention.

Specific examples of compositions, which are constituted as a 1% solution in water, freeze dried and may be made up by adding 0.9% aqueous sodium chloride solution to give the required concentration, preferably 1mg-10mg/ml, are as follows:

### Composition 1

- Compound of Example 1: 50mg

### Composition 2

- Compound of Example 1: 50mg
- Glycine: 31mg

Further specific examples of compositions are as above, but where the compound of example 1 is replaced by any one of examples 2 to 5.

The pharmaceutical compositions of the invention will normally be administered to man in order to combat infections caused by bacteria, in the same general manner as that employed for imipenem due allowance being made in terms of dose levels for the pharmacokinetics of the compound of the present invention relative to the clinical use of imipenem. Thus each patient will receive a daily intravenous, subcutaneous or intramuscular dose of 0.05 to 5g, and preferably 0.1 to 2.5g, of the compound of this invention, the composition being administered 1 to 4 times per day, preferably 1 or 2 times a day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose. Thus a suitable daily oral dose is 0.05 to 5g of the compound of this invention, the composition being administered 1 to 4 times per day.

In a further aspect the present invention provides a process for preparing the compounds of the formula (I) or a pharmaceutically acceptable salt or in vivo hydrolysable ester thereof which process comprises deprotecting a compound of the formula (V) wherein A is optionally further substituted as in formula (I): 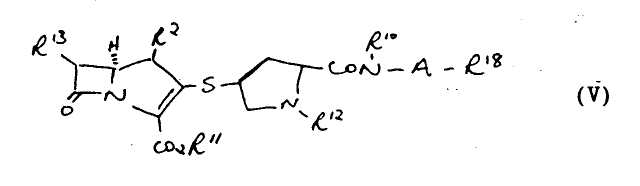 wherein A is as hereinbefore defined; R² is as hereinbefore defined; R¹⁰ is a group R³ or an amino protecting group; R¹³ is a group R¹, protected hydroxymethyl or 1-(protected hydroxy)ethyl; R¹¹ is hydrogen or a carboxy protecting group; R¹² is hydrogen or an amino protecting group, R¹⁸ is carboxy or a protected carboxy group and wherein any optional substituent on the heteroaryl ring is optionally protected; and wherein at least one protecting group is present; and thereinafter if necessary;
(i) forming a pharmaceutically acceptable salt,
(ii) esterifying to form an in vivo hydrolysable ester.

Protecting groups may in general be chosen from any of the groups described in the literature or known to the skilled chemist as appropriate for the protection of the group in question, and may be introduced by conventional methods.

Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

The compounds of the formula (V) are novel and form another aspect of the invention.

Specific examples of protecting groups are given below for the sake of convenience, in which "lower" signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned is of course within the scope of the invention.

A carboxy protecting group may be the residue of an ester-forming aliphatic or araliphatic alcohol or of an ester-forming silanol (the said alcohol or silanol preferably containing 1-20 carbon atoms).

Examples of carboxy protecting groups include straight or branched chain (1-12C)alkyl groups (eg isopropyl, t-butyl); lower alkoxy lower alkyl groups (eg methoxymethyl, ethoxymethyl, isobutoxymethyl); lower aliphatic acyloxy lower alkyl groups, (eg acetoxymethyl, propionyloxymethyl, butyryloxymethyl, pivaloyloxymethyl); lower alkoxycarbonyloxy lower alkyl groups (eg 1-methoxycarbonyloxyethyl, 1-ethoxycarbonyloxyethyl); aryl lower alkyl groups (eg p-methoxybenzyl, o-nitrobenzyl, p-nitrobenzyl, benzhydryl and phthalidyl); tri(lower alkyl)silyl groups (eg trimethylsilyl and t-butyldimethylsilyl); tri(lower alkyl)silyl lower alkyl groups (eg trimethylsilylethyl); diaryl(lower alkyl)silyl groups (eg t-butyldiphenylsilyl); and (2-6C)alkenyl groups (eg allyl and vinylethyl).

Methods particularly appropriate for the removal of carboxyl protecting groups include for example acid-, base-, metal- or enzymically-catalysed hydrolysis, for groups such as p-nitrobenzyloxycarbonyl, hydrogenation and for groups such as o-nitrobenzyloxycarbonyl, photolytically.

Examples of hydroxy protecting groups include lower alkenyl groups (eg allyl); lower alkanoyl groups (eg acetyl); lower alkoxycarbonyl groups (eg t-butoxycarbonyl); lower alkenyloxycarbonyl groups (eg allyloxycarbonyl); aryl lower alkoxycarbonyl groups (eg benzoyloxycarbonyl, p-methoxybenzyloxycarbonyl, o-nitrobenzyloxycarbonyl, p-nitrobenzyloxycarbonyl); tri lower alkylsilyl (eg trimethylsilyl, t-butyldimethylsilyl); diaryl(lower alkyl)silyl (eg t-butyldiphenylsilyl) and aryl lower alkyl (eg benzyl) groups.

Examples of amino protecting groups include formyl, aralkyl groups (eg benzyl and substituted benzyl, eg p-methoxybenzyl, nitrobenzyl and 2,4-dimethoxybenzyl, and triphenylmethyl); di-p-anisylmethyl and furylmethyl groups; lower alkoxycarbonyl (eg t-butoxycarbonyl); lower alkenyloxycarbonyl (eg allyloxycarbonyl); aryl lower alkoxycarbonyl groups (eg benzyloxycarbonyl, p-methoxybenzyloxycarbonyl, o-nitrobenzyloxycarbonyl, p-nitrobenzyloxycarbonyl); trialkylsilyl (eg trimethylsilyl and t-butyldimethylsilyl); diaryl(lower alkyl)silyl (eg t-butyldiphenylsily); alkylidene (eg methylidene); benzylidene and substituted benzylidene groups.

Methods appropriate for removal of hydroxy and amino protecting groups include, for example, acid-, base-, metal- or enzymically-catalysed hydrolysis, for groups such as p-nitrobenzyloxycarbonyl, hydrogenation and for groups such as o-nitrobenzyloxycarbonyl, photolytically.

In another aspect of the present invention the compounds of the formulae (I) and (V) may be prepared by
a) reacting compounds of the formulae (VI) and (VII): 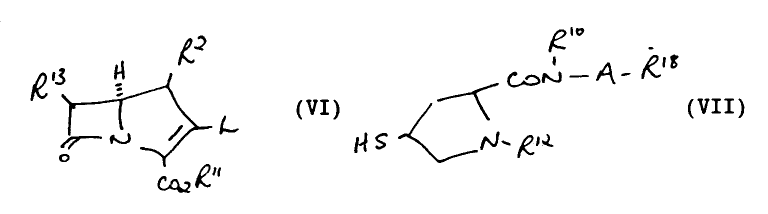 wherein A, R², R¹⁰, R¹¹, R¹², R¹³ and R¹⁸ are as hereinbefore defined, optional substituents on A are as hereinbefore defined and L is a leaving group, or
b) cyclising a compound of the formula (VIII): 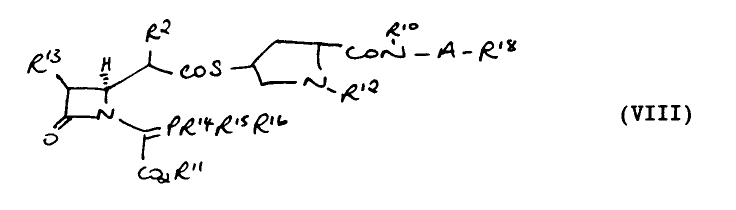 wherein A, R², R¹⁰, R¹¹, R¹², R¹³ and R¹⁸ are as hereinbefore defined, optional substituents on A are as hereinbefore defined and R¹⁴, R¹⁵ and R¹⁶ are independently selected from C₁₋₆alkoxy, aryloxy, di-C₁₋₆alkylamino and diarylamino or any two of R¹⁴-R¹⁶ represent o-phenylenedioxy or one of R¹⁴-R¹⁶ is C₁₋₄alkyl, allyl, benzyl or phenyl and the other two values are independently selected from C₁₋₄alkyl, trifluoromethyl or phenyl wherein any phenyl- group is optionally substituted with C₁₋₃alkyl or C₁₋₃alkoxy; and wherein any functional group is optionally protected and thereinafter if necessary:
   (i) removing any protecting groups;
   (ii) forming a pharmaceutically acceptable salt;
   (iii) esterifying to form an in vivo hydrolysable ester.

Suitably in the compound of the formula (VI), L is the reactive ester of a hydroxy group such as a sulphonate (for example C₁₋₆alkanesulphonyloxy, trifluoromethanesulphonyloxy, benzenesulphonyloxy, toluenesulphonyloxy), a phosphoric ester (for example a diarylphosphoric ester such as diphenylphosphoric ester) or L is a halide (for example chloride). In an alternative L is a sulphoxide for example -SOCH=CH-NHCOCH₃ which may be readily displaced. Preferably L is diphenylphosphoric ester (-OP(O)(OPh)₂).

Compounds of the formula (VI) and their preparation are well known in the carbapenem literature, for example see EP-A-126587, EP-A-160391, EP-A-243686 and EP-A-343499.

The reaction between the compounds of the formulae (VI) and (VII) is typically performed in the presence of a base such as an organic amine for example di-isopropylethylamine or an inorganic base for example an alkali metal carbonate such as potassium carbonate. The reaction is conveniently performed at a temperature between -25°C and ambient, suitably at about 0°C. The reaction is generally performed in an organic solvent such as acetonitrile or dimethylformamide. The reaction is generally performed in a manner similar to that described in the literature for similar reactions.

The compounds of the formula (VII) are novel and form another aspect of the present invention.

The compounds of the formula (VII) may be prepared by the deprotection of a compound of the formula (IX): 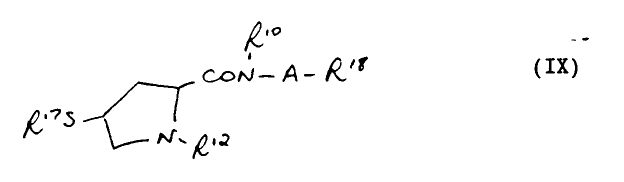 wherein A, R¹⁰, R¹² and R¹⁸ are as hereinbefore defined, optional substitutents on A are as hereinbefore defined and R¹⁷ is a protecting group, for example C₁₋₆alkanoyl or C₁₋₆alkoxycarbonyl. Preferred values for R¹⁷ are acetyl and t-butoxycarbonyl. The compounds of the formula (IX) can be converted to the compounds of the formula (VII) by standard methods of deprotection, for example acetyl groups can be removed by basic hydrolysis in aqueous alkanol, alkenol or cyclic ether, for example ethanol, dioxane or tetrahydrofuran.

The compounds of the formula (IX) are novel and form another aspect of the present invention.

The compounds of the formula (IX) may be prepared by the reaction of an activated derivative of a compound of the formula (X), which may be formed in situ, with a compound of the formula (XI): 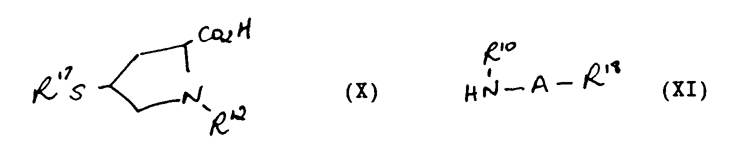 wherein A, R¹⁰, R¹², R¹⁷ and R¹⁸ are as hereinbefore defined and optional substitutents on A are as hereinbefore defined. Activated derivatives of the compound of the formula (X) include acid halides, anhydrides and 'activated' esters such as 1H-benzol-1,2,3-triazol-1-yl, pentafluorophenyl and 2,4,5-trichlorophenyl esters or the benzimidazol-2-yl ester of the thiocarboxylic acid corresponding to (X). The reaction of an activated derivative of a compound of the formula (X) and a compound of the formula (XI) is performed under standard methods, for example in dichloromethane at 4°C in the presence of diisopropylethylamine or in chloroform at ambient temperature in the presence of N-ethoxycarbonyl-2-ethoxy-1,2-dihydroquinoline.

The compounds of the formulae (X) and (XI) are prepared by standard methods known to the skilled chemist such as the methods of the Examples hereinafter, the methods described in EP-A-126587 or by methods analogous or similar thereto.

Suitably, in the compounds of the formula (VIII), R¹⁴, R¹⁵ and R¹⁶ are independently selected from C₁₋₆ alkoxy such as methoxy, ethoxy, isopropoxy, n-propoxy or n-butoxy; aryloxy such as optionally phenoxy; di-C₁₋₆alkylamino such as dimethylamino or diethylamino; diarylamino such as diphenylamino or any two of R¹⁴-R¹⁶ represent o-phenylenedioxy. Preferably each of R¹⁴-R¹⁶ have the same value and are C₁₋₆alkoxy for example methoxy, ethoxy, isopropoxy or n-butoxy or are phenoxy.

The compounds of the formula (VIII) are cyclized under conventional conditions known in the art to form compounds of the formula (V). Typical conditions are heating in a substantially inert organic solvent such as toluene, xylene or ethyl acetate at temperatures in the region 60-150°C. Typically the reaction is performed in an atmosphere of nitrogen and is carried out in the presence of a radical scavenger for example hydroquinone.

The compounds of the formula (VIII) may be formed and cyclized in situ. The compounds of the formula (VIII) may conveniently be prepared by reacting compounds of the formulae (XII) and (XIII):

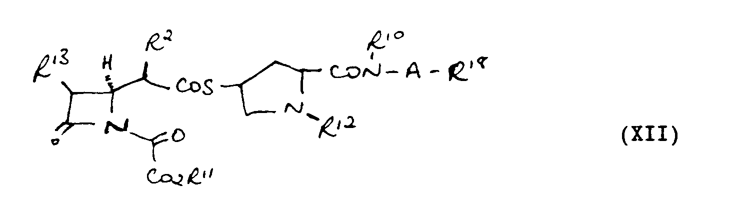

PR¹⁴R¹⁵R¹⁶ (XIII)

wherein A, R², R¹⁰, R¹¹-R¹⁶, and R¹⁸ are as hereinbefore defined and optional substituents on A are as hereinbefore defined. Suitably the compound of the formula (XIII) is a phosphite or is the functional equivalent of such a compound.

The reaction between the compounds of the formulae (XII) and (XIII) is conveniently performed in an organic solvent such as toluene, xylene, ethyl acetate, chloroform, dichloromethane, acetonitrile or dimethylformamide. Typically the reaction is carried out at an elevated temperature for example 60-150°C.

The compounds of the formula (XII) may be prepared by a number of methods known in the art. For example the compounds of the formula (XII) may be prepared by the acylation of a compound of the formula (XIV): 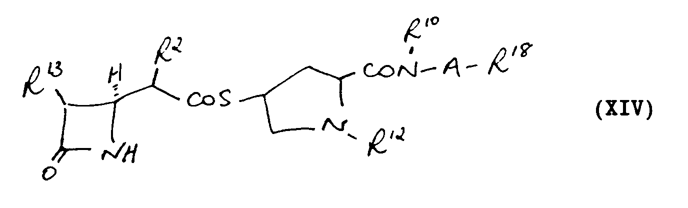 wherein A, R², R¹⁰, R¹², R¹³, and R¹⁸ are as hereinbefore defined and optional substituents on A are as hereinbefore defined with a compound of the formula (XV):

Cl-CO-COOR¹¹ (XV)

wherein R¹¹ is as hereinbefore defined.

The compounds of the formula (XIV) may be prepared by reacting compounds of the formulae (XVI) and (VII): 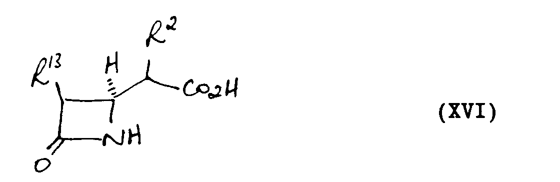 wherein R² and R¹³ are as hereinbefore defined. The compounds of the formula (XVI) are known in the art and may be reacted with the compounds of the formula (VII) under conventional acylation methods known in the art.

Compounds of the formulae (VII), (XII) and (XIV) are novel and, as such, form another aspect of this invention.

The following biological test methods, data and Examples serve to illustrate the present invention.

### Antibacterial Activity

The pharmaceutically acceptable carbapenem compounds of the present invention are useful antibacterial agents having a broad spectrum of activity in vitro against standard laboratory microorganisms, both Gram-negative and Gram-positive, which are used to screen for activity against pathogenic bacteria. The antibacterial spectrum and potency of a particular compound may be determined in a standard test system. In particular the carbapenems of the present invention show good stability to beta-lactamases and in general particularly good pharmacokinetics, especially as regards half life. In general compounds show significant improvement over imipenem.

The antibacterial properties of the compounds of the invention may also be demonstrated in vivo in conventional tests.

Carbapenem compounds have generally been found to be relatively non-toxic to warm-blooded animals, and this generalisation holds true for the compounds of the present invention. Compounds representative of the present invention were administered to mice at doses in excess of those required to afford protection against bacterial infections, and no overt toxic symptoms or side effects attributable to the administered compounds were noted.

The following results were obtained for representative compounds on a standard in vitro test system using Diagnostic Sensitivity Test. The antibacterial activity is described in terms of the minimum inhibitory concentration (MIC) determined by the agar-dilution technique with an inoculum size of 10⁴ CFU/spot.

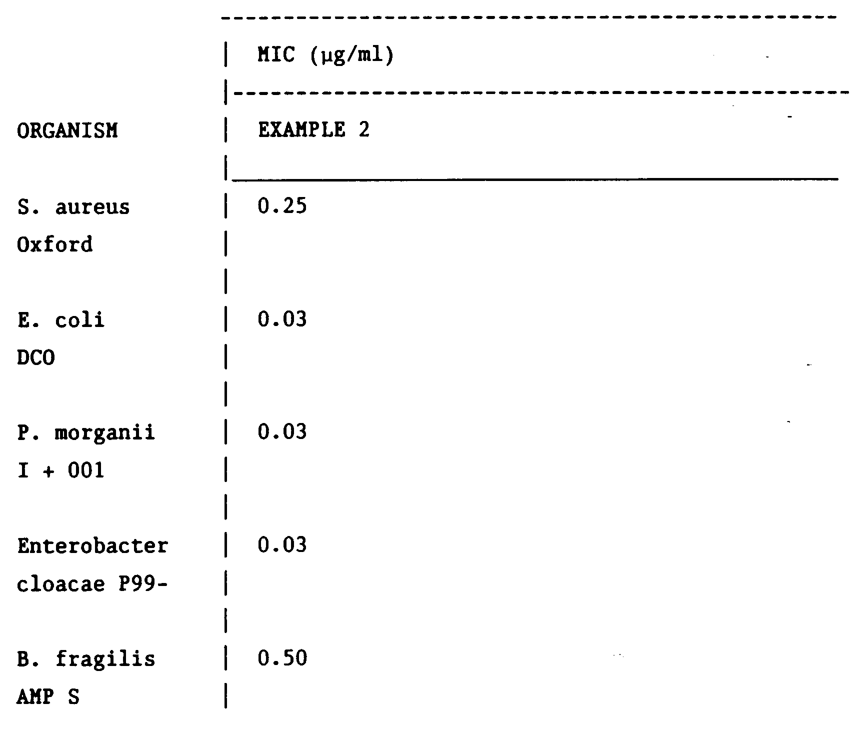

In the following examples, which are representative of the scope:
(a) NMR spectra were taken at 200MHz or 400MHz in DMSO-d₆/CDCOOD unless otherwise stated;
(b) allyloxy means the propen-1-yloxy group -OCH₂CH=CH₂;
(c) THF means tetrahydrofuran;
(d) DMF means dimethylformamide;
(e) DMSO means dimethylsulphoxide;
(f) EEDQ means N-ethoxycarbonyl-2-ethoxy-1,2-dihydroquinoline
(g) evaporation of solvents was carried out under reduced pressure;
(h) HPLC means high pressure liquid chromatography;
(i) temperatures are in degrees centigrade; and
(j) Meldrum's acid means 2,2-dimethyl-1,3-dioxane-4,6-dione

### Example 1

### (1R,5S,6S,8R,2'S,4'S)-2-(2-(5-Carboxy-2-thiazolylcarbamoyl)pyrrolidin-4-ylthio)-6-(1-hydroxyethyl)-1-methylcarbapenem-3-carboxylic acid, disodium salt.

To a solution of allyl (1R,5S,6S,8R,2'S,4'S)-2-(1-(4-nitrobenzyloxycarbonyl)-2-(5-carboxy-2-thiazolylcarbamoyl) pyrrolidin-4-ylthio)-6-(1-hydroxyethyl)-1-methylcarbapenem-3-carboxylate, diisopropylethylamine salt (640mg; contaminated by an unknown amount of tri-n-butylphosphine) in DMF at 50°C was added Meldrum's acid (100 mg, 0.7 mmol) followed by tetrakis-(triphenylphosphine)palladium (60 mg, 0.05 mmol). The reaction mixture was stirred at 50°C for 10 minutes, then cooled to ambient temperature and diluted with a solution of 1M phosphate buffer (10 ml). Zinc powder (1 g) was added in small portions over 45 minutes to the stirred solution. After 1 hour, at ambient temperature, the undissolved substances were removed by filtration over diatomaceous earth and the pH of the filtrate adjusted to 8.0 with solid sodium hydrogen carbonate. The reaction mixture was filtered, concentrated under reduced pressure and the resulting residue purified by reverse phase chromatography (Nucleosil C18, 3.5 x 20 cm), with water as eluant, to give a mixture of the title compound and phosphate buffer after freeze drying. The mixture was purified a second time under the same conditions to give the title compound (72 mg, 30%) as a foam after freeze drying.
NMR : δ 1.16 (d, 3H); 1.18 (d, 3H); 1.76-1.83 (m, 1H); 2.61-2.69 (m, 1H); 2.78-2.83 (m, 1H); 3.20 (dd, 1H); 3.37-3.42 (m, 2H); 3.66-3.70 (m, 1H); 3.95-3.79 (m, 1H); 4.06-4.10 (m, 1H); 4.16 (dd, 1H); 7.97 (s, 1H).
MS (+ve FAB): 482 MH⁺; 527 MH⁺ Na salt; 550 MH⁺ diNa salt.
The starting material was prepared as follows:

### (2S,4S) 1-(4-Nitrobenzyloxycarbonyl)-2-(5-carboxy-2-thiazolylcarbamoyl) pyrrolidin-4-ylthioacetate.

(2S,4S)-1-(4-Nitrobenzyloxycarbonyl)-4-acetylthio-2-carboxypyrrolidine (368 mg, 1 mmol) was solubilized at ambient temperature in thionyl chloride (3 ml). The mixture was stirred for 3 hours at ambient temperature and thionyl chloride evaporated. The residual oil was taken up in toluene, the solvent evaporated and the residue dried under reduced pressure. The crude acid chloride was dissolved in dichloromethane (10 ml) and added dropwise to a solution of ethyl 2-aminothiazol-5-carboxylate (O. Dann, Ber. Dtsch. Chem. Ges. 1943, 76, 419) (172 mg, 1 mmol) and N-ethyldiisopropylamine (0.2 ml, 1.15 mmol) in dichloromethane (10 ml). The reaction mixture was stirred for 2 hours at ambient temperature and overnight at 4°C. After evaporation to dryness, the crude material was purified by subjecting to flash chromatography on silica, eluting with dichloromethane/ethyl acetate (6:4) to give the title compound as a yellow foam (430 mg, 82%).
NMR: δ 1.31 (m, 3H); 1.95 (m, 1H); 2.33 (s, 3H); 2.80 (m, 1H); 3.38 (m, 1H): 4.01-4.13 (m, 2H); 4.29 (m, 2H); 4.63 (m, 1H); 5.02-5.30 (m, 2H); 7.43 (d, 1H); 7,65 (d, 1H); 7.96 (d, 1H); 8.11 (d, 1H); 8.23 (d, 1H).

### (2S,4S)-1-(4-Nitrobenzyloxycarbonyl)-2-(5-carboxy-2-thiazolylcarbamoyl)pyrrolidin-4-ylthiol.

(2S,4S)-1-(4-Nitrobenzyloxycarbonyl)-2-(5-carboxy-2-thiazolyl-carbamoyl)pyrrolidin-4-ylthioacetate was solubilised in a mixture of dioxane/H₂O (1/1; 4 ml) and treated with a 2M aqueous solution of NaOH (1.5 ml, 3 mmol). The reaction mixture was stirred for 6 hours at ambient temperature and kept overnight, at 4°C. A 2M aqueous solution of NaOH (0.3 ml, 0.6 mmol) was added to the reaction and stirring continued for 5 hours at ambient temperature. After evaporation to dryness, the crude reaction mixture was dissolved in water and acidified with 1M HCl to pH 4.5. DMF was added to the resulting suspension until a clear solution was obtained. This mixture was further purified by subjecting to chromatography on HP2OSS resin with a gradient of acetonitrile (0-60%) in water to give the expected thiol (145 mg, 40%) and a small amount of the corresponding disulfide (35 mg, 10%).
NMR: δ 2.00 (m, 1H); 2.79 (m, 1H); 3.24-3.72 (m, 2H); 3.99 (m, 1H); 4.56 (m, 1H); 5.00-5.27 (m, 2H); 7.43 (d, 1H); 7.65 (d, 1H); 7.90-7.97 (m, 2H); 8.23 (d, 1H).

Allyl (1R,5R,6S,8R)-6-(1-hydroxethyl)-1-methyl-2-diphenyl-phosphoryloxycarbapenem-3-carboxylate was prepared as follows:

To a solution of allyl (1R,5R,6S,8R)-6-(1-hydroxyethyl)-1-methyl-2-oxocarbapenem-3-carboxylate [prepared in situ from allyl 2-diazo-3-oxo-4-(R)-methyl-4-[(3S,4R)-3-(1-(R)-hydroxyethyl)-2-oxoazetidin-4-yl]-butanoate and rhodium octanoate: see for example EP-A-208889] and di-isopropylethylamine (1.1 equivalents) in acetonitrile, at 0°C, under an argon atmosphere, was added dropwise diphenyl chlorophosphate (1.1 equivalents). The solution was stirred at ambient temperature for 30 minutes to form the corresponding 2-diphenylphosphoryloxycarbapenem.

### Allyl (1R,5S,6S,8R,2'S,4'S)-2-(1-(4-nitrobenzyloxycarbonyl)-2-(5-carboxy-2-thiazolylcarbamoyl)pyrrolidin-4-ylthio)-6-(1-hydroxyethyl)-1-methylcarbapenem-3-carboxylate, diisopropylethylamine salt.

To a solution of allyl (1R,5S,6S,8R) 6-(1-hydroxyethyl)-1-methyl-2-diphenylphosphoryloxycarbapenem-3-carboxylate (250 mg, 0.5 mmol) in DMF (3 ml), at 0°C, were added sequentially diisopropylethylamine (0.21 ml, 1.2 mmol), a solution containing a mixture of (2S,4S)-1-(4-nitrobenzyloxycarbonyl)-2-(5-carboxy-2-thiazol-carbamoyl)pyrrolidin-4-ylthiol (150 mg, 0.33 mmol) and the corresponding disulfide (45 mg, 0.05 mmol) in DMF (3 ml), tri-n-butylphosphine (0.14 ml, 0.56 mmol) and 2 drops of water. The reaction mixture was stirred for 1 hour at ambient temperature and directly purified by subjecting to chromatography on HP20SS (100 ml) with a gradient of acetonitrile (0-60%) in water. The title compound contaminated with tri-n-butylphosphine eluted between 20% and 45% acetonitrile and was obtained, after lyophilisation, as an orange gum (645 mg). This was used without further purification.
NMR: δ 1.17 (m, 6H); 1.27 (m, 14H); 1.90 (m, 1H); 2.82 (m, 1H); 3.14 (q, 2H); 3.26 (dd, 1H); 3.38 (m, 1H); 3.55 (dd, 1H); 3.63 (m, 2H); 3.97-4.24 (m, 3H); 4.58-4.70 (m, 2H); 5.02-5.43 (m, 4H); 5.90 (m, 1H); 7.44 (d, 1H); 7.66 (d, 1H); 7.95-8.02 (m, 2H); 8.23 (d, 1H).

### Example 2

### (1R,5S,6S,8R,2'S,4'S)-2-(2-(4-Carboxy-2-thiazolylcarbamoyl)pyrrolidin-4-ylthio)-6-(1-hydroxyethyl)-1-methylcarbapenem-3-carboxylic acid, disodium salt.

To a solution of allyl (1R,5S,6S,8R,2'S,4'S)-2-(1-nitrobenzyloxycarbonyl)-2-(4-carboxy-2-thiazolylcarbamoyl)pyrrolidin-4-ylthio)-6-(1-hydroxyethyl)-1-methylcarbapenem-3-carboxylate (250 mg, 0.35 mmol) in DMF (10 ml) was added Meldrum's acid (102 mg, 0.70 mmol) followed by tetrakis(triphenylphosphine)palladium (41 mg, 0.035 mmol). The reaction mixture was stirred at 40°C for 30 minutes, cooled to ambient temperature and diluted with aqueous solution of 1M phosphate buffer (10 ml). Zinc powder (274 mg, 4.2 mmol) was then added in small portions to the mixture. After 2 hours at ambient temperature, a saturated aqueous solution of sodium hydrogen carbonate was added to the reaction mixture to adjust the pH of the solution to 7.7, the solid filtered off over diatomaceous earth and the filtrate concentrated. The residue was purified by subjecting to reverse phase chromatography (Nucleosil C18, 3.5 x 20 cm) with acetonitrile in water (0:100 and 4:96) as eluant to give, after freeze drying, the title compound as a white foam (38 mg, 23%).
NMR: δ 1.16 (m, 6H); 1.75 (m, 1H); 2.61 (m, 1H); 2.75 (m, 1H); 3.20 (dd, 1H); 3.37 (m, 1H); 3.39 (m, 1H); 3.64 (m, 1H); 3.97 (m, 1H); 4.01 (m, 1H); 4.16 (dd, 1H); 7.92 (s, 1H).
MS (-ve FAB): 481 MH-
The starting material was prepared as follows:

### (2S,4S) 1-(4-Nitrobenzyloxycarbonyl)-2-(4-carbethoxy-2-thiazolyl carbamoyl)pyrrolidin-4-ylthioacetate.

To a solution of (2S,4S)-1-(4-nitrobenzyloxycarbonyl)-4-acetylthio-2-carboxypyrrolidine (4.7 g; 12.8 mmol) in chloroform (250 ml) was added EEDQ (3.5 g; 14 mmol) followed by 2-amino-4-carbethoxythiazole. [J.M. Sprague, R.M. Lincoln and C. Ziegler, J. Amer. Chem. Soc. 68, 266 (1946)] (2.2g, 12.8 mmol). The reaction mixture was stirred at ambient temperature overnight, the solvent evaporated and the residue taken up in DMF (15 ml). This solution was purified by subjecting to chromatography on HP20SS resin, with a gradient of acetonitrile (0-50%) in water. Concentration in vacuo gave an oil which was separated from the aqueous phase, taken up in dichloromethane, dried over MgSO₄, filtered and the solvent evaporated to give the title compound as a foam (4.3 g, 65%).
NMR: δ 1.30 (t, 3H); 1.95 (m, 1H); 2.80 (m, 1H); 3.40 (m, 1H); 3.95-4.15 (m, 2H); 4.28 (q, 2H); 4.55 (m, 1H); 5.00-5.30 (m, 2H); 7.43 (d, 1H); 7.66 (d, 1H); 7.93 (d, 1H); 8.05 (d, 1H); 8.24 (d,1H).

### (2S,4S)-1-(4-Nitrobenzyloxycarbonyl)-2-(4-carboxy-2-thiazolylcarbamoyl)pyrrolidin-4-ylthiol.

To a solution of (2S,4S)-1-(4-nitrobenzyloxycarbonyl)-2-(4-carbethoxy-2-thiazolylcarbamoyl)pyrrolidin-4-ylthioacetate (3.1 g, 6 mmol) in ethanol (120 ml), at ambient temperature, was added a 1M aqueous solution of sodium hydroxide (22 ml, 22 mmol). After stirring at ambient temperature for 17 hours, ethanol was evaporated, the residue taken up in water (80 ml) and the pH of the solution adjusted to 4.5 with a 1M aqueous solution of hydrochloric acid. The precipitate was filtered off, washed with water, dissolved in DMF (40 ml) and mixed with tri-n-butyl-phosphine (2 ml) and water (0.8 ml). This mixture was then purified by subjecting to chromatography on HP20SS resin with a gradient of acetonitrile (0-40%) in water. Partial evaporation of the solvents and freeze drying gave the title compound as a white foam (2.06 g, 76%).
NMR: δ 1.76 (m, 1H); 2.75 (m, 1H); 3.15-3.59 (m, 2H); 4.00 (m, 1H); 4.50 (m, 1H); 5.03-5.28 (m, 2H); 7.43 (d, 1H); 7.66 (d, 1H); 7.93 (d, 1H); 7.94 (d, 1H); 8.24 (d, 1H).

### Allyl (1R,5S,6S,8R,2'S,4'S) 2-(1-(4-nitrobenzyloxycarbonyl 2-(4-carboxy-2-thiazolylcarbamoyl)pyrrolidin-4-ylthio)-6-(1-hydroxyethyl)-1-methylcarbapenem-3-carboxylate.

To a solution of allyl (1R,5S,6S,8R) 6-(1-hydroxyethyl)-1-methyl-2-diphenylphosphonyloxycarbapenem-3-carboxylate (552 mg, 1.1 mmol) in DMF (16 ml) was added sequentially at 0°C N-diispropylethylamine (0.46 ml, 2.6 mmol), (2S, 4S)-1-(4-nitrobenzyloxycarbonyl)-2-(4-carboxy-2-thiazolylcarbamoyl)pyrrolidin-4-ylthiol (500 mg, 1.1 mmol), tri-n-butylphosphine (0.33 ml, 1.3 mmol) and water (0.1 ml). The reaction mixture was stirred at 0°C for 2.5 hours and purified by subjecting to chromatography on HP20SS resin with a gradient of acetonitrile (0-42%) in water. Partial evaporation of the solvents and freeze drying gave the title compound as a white foam (250 mg, 32%).
NMR: δ 1.23 (m, 6H); 1.96 (m, 1H); 2.88 (m, 1H); 3.32 (m, 1H); 3.45 (m, 1H); 3.63 (m, 1H); 4.02-4.06 (m, 2H); 4.25 (m, 1H); 4.31 (m, 1H); 4.60-4.76 (m, 3H); 5.08-5.49 (m, 4H); 5.95 (m, 1H); 7.51 (d, 1H); 7.74 (d, 1H); 8.01 (d, 1H); 8.06 (d, 1H); 8.31 (d, 1H).

### Example 3

### (1R,5S,6S,8R,2'S,4'S)-2-(2-(5-Carboxy-1,3,4-thiadiazol-2-carbamoyl) pyrrolidin-4-ylthio)-6-(1-hydroxyethyl)-1-methylcarbapenem-3-carboxylic acid, dipotassium salt.

To a solution containing a 2:1 mixture (700 mg) of allyl (1R,5S,6S,8R,2'S,4'S)-2-(1-(4-nitrobenzyloxycarbonyl)-2-(5-carboxy-1,3,4-thiadiazol-2-carbamoyl)pyrrolidin-4-ylthio)-6-(1-hydroxyethyl)-1-methylcarbapenem-3-carboxylate and an impurity in a mixture of DMF (5 ml) and ethyl acetate (5 ml) were added Meldrum's acid (100 mg, 0.69 mmol) and tetrakis(triphenylphosphine)palladium (40 mg, 0,035 mmol). After 1 hour at ambient temperature, the solvents were evaporated, the residue taken up in water (40 ml) and potassium carbonate (138 mg, 1 mmol) added to the solution. This mixture was washed with ethyl acetate, the aqueous phase mixed with ethyl acetate (40 ml) and 10% palladium on charcoal (300 mg) and stirred under a hydrogen atmosphere (30 psi) at ambient temperature for 1 hour. The reaction mixture was filtered through diatomaceous earth, partially concentrated and purified by subjecting to reverse phase chromatography (Nucleosil C18, 3.5 x 20 cm) with acetonitrile in water as eluent. The title compound was eluted with water and obtained after freeze drying as a white foam (72 mg, 19%).
NMR: δ 1.15 (m, 6H); 1.76 (m, 1H); 2.64 (m, 1H); 2.80 (m, 1H); 3.20 (m, 1H); 3.35-3.41 (m, 2H); 3.67 (m, 1H); 3.97 (m, 1H); 4.07 (m, 1H); 4.16 (m, 1H).
The starting material was prepared as follows:

### (2S,4S)-1-(4-Nitrobenzyloxycarbonyl)-2-(5-carbethoxy-1,3,4-thiadiazol-2-carbamoyl)pyrrolidin-4-ylthioacetate.

To a solution of (2S,4S)-1-(4-nitrobenzyloxycarbonyl)-4-acetylthio-2-carboxypyrrolidine (1.83 g, 5 mmol) in chloroform (50 ml) were added EEDQ (1.35 g, 5.5. mmol) and ethyl 2-amino-1,3,4-thiadiazol-5-carboxylate (G. Weber and F Maggio, Ann. Chim. (Rome) 49, 2124 (1959); CA 54 16648d). After 1 hour at ambient temperature the solvent was evaporated, the residue mixed with ethanol (5 ml) and diethyl ether (50 ml) to give the title compound as a crystalline solid (1-90 g, 73%).
NMR: δ 1.35 (td, 3H); 1.95 (m, 1H); 2.33 (d, 1H); 2.80 (m, 1H); 3.40 (m, 1H); 3.97-4.11 (m, 2H); 4.40 (q, 2H); 4.64 (m, 1H); 5.00-5.29 (m, 2H); 7.42 (d, 1H); 7.65 (d, 1H); 7.97 (d, 1H); 8.24 (d, 1H).

### (2S,4S)-1-(4-Nitrobenzyloxycarbonyl)-2-(5-carboxy-1,3,4-thiadiazol-2-carbamoyl)pyrrolidin-4-ylthiol.

To a suspension of (2S,4S)-1-(4-nitrobenzyloxycarbonyl)-2-(5-carbethoxy-1,3,4-thiadiazol-2-carbamoyl)pyrrolidin-4-ylthioacetate in water (2.0 ml) was added, at ambient temperature, a 1M aqueous solution of sodium hydroxide (8 ml). After the addition the reaction mixture was neutralised with acetic acid (0.5 ml) and purified by subjecting to chromatography on HP20SS resin with a gradient of acetonitrile (0.35%) in water. Freeze drying gave the title compound as a foam (0.89 g, 76%).
NMR: δ 1.89 (m, 1H); 2.76 (m, 1H); 3.25-3.43 (m, 2H); 4.01 (m, 1H); 4.56 (m, 1H); 5.06-5.25 (m, 2H); 7.44 (d, 1H); 7.66 (d, 1H); 8.01 (d, 1H); 8.24 (d, 1H).

### Allyl (1R,5S,6S,8R,2S',4S')-2-(1-(4-nitrobenzyloxycarbonyl)-2-(5-carboxy-1,3,4-thiadiazol-2-carbamoyl)pyrrolidin-4-ylthio)-6-(1-hydroxyethyl)-1-methylcarbapenem-3-carboxylate.

To a solution of allyl (1R,5S,6S,8R) 6-(1-hydroxyethyl)-1-methyl-2-diphenylphosphoryloxycarbapenem-3-carboxylate (0.8 g, 1.6 mmol) in DMF (10 ml) was added sequentially, at ambient temperature, N-diisopropylethylamine (1.2 ml, 6.9 mmol), (2S,4S)-1-(4-nitrobenzyloxycarbonyl)-2-(5-carboxy-1,3,4-thiadiazol-2-carbamoyl) pyrrolidin-4-ylthiol (0.72 g, 1.6 mmol), tri-n-butylphosphine (0.4 ml, 1.6 mmol) and 3 drops water. The reaction mixture was stirred for 2 hours at ambient temperature time and further purified by subjecting to chromatography on HP20SS resin, using a gradient of acetonitrile (0-40%) in water. Freeze drying gave a mixture of the title compound and an impurity. This mixture was used in the deprotection step.

### Example 4

### (1R,5S,6S,8R,2'S,4'S)-2-(2-(2-Carboxy-1H-imidazol-4-ylcarbamoyl)pyrrolidin-4-ylthio)-6-(1-hydroxyethyl)-1-methylcarbapenem-3-carboxylic acid, disodium salt.

A solution of 4-nitrobenzyl (1R,5S,6S,8R,2'S,4'S)-2-(1-(4-nitrobenzyloxycarbonyl)-2-(2-carboxy-1H-imidazol-4-ylcarbamoyl)-pyrrolidin-4-ylthio)-6-(1-hydroxyethyl)-1-methylcarbapenem-3-carboxylate, N-diisopropylethylamine salt (300 mg, 0.38 mmol) in a mixture of ethyl acetate (12 ml) and water (12 ml) was mixed with sodium hydrogen carbonate (110 mg, 1.3 mmol) and 10% palladium on charcoal (200 mg). The mixture was stirred under a hydrogen atmosphere (30 psi) for 30 minutes. The catalyst was filtered off, the organic phase discarded and the aqueous phase partially concentrated and purified by subjecting to reverse phase chromatography (Nucleosil C18, 3.5 x 20 cm) with water as eluant to give, after freeze drying, the title compound (57 mg, 23%).
NMR: δ 1.21 (d, 3H); 1.23 (d, 3H); 1.76 (m, 1H); 2.75 (m, 1H); 2.87 (m, 1H); 3.27 (dd, 1H); 3.45 (m, 1H); 3.45 (m, 1H); 3.54 (m, 1H); 3.75 (m, 1H); 4.00-4.10 (m, 2H); 4.22 (dd, 1H); 7.31 (s, 1H).
MS (+ve FAB): 460 MH⁺
The starting material was prepared as follows:

### 4-Amino-1H-imidazol-2-carboxylic acid

A solution of ethyl 4-amino-1H-imidazol-2-carboxylate (2.0 g, 12.9 mmol) (E. Gomez, C. Avendano and A. McKillop, Tetrahedron 1986, 42, 2635) in ethanol (50 ml) and 1M aqueous sodium hydroxide (14 ml) was refluxed for 20 minutes. The reaction mixture was concentrated to 20 ml and the pH of the solution adjusted to 5 with concentrated hydrogen chloride. The solid which precipitated, collected by filtration and washed with ethanol and ether to give the title compound (1.5 g, 32%).
NHR: δ 7.07 (s, 1H).

### (2S,4S)-1-(4-Nitrobenzyloxycarbonyl)-2-(2-carboxy-1H-imidazol-4-ylcarbamoyl)pyrrolidin-4-ylthio.

To a solution of (2S,4S)-1-(4-nitrobenzyloxycarbonyl)-4-acetylthio-2-carboxypyrrolidine (1.83 g, 5 mmol) in chloroform (20 ml) was added EEDQ (1.35 g, 5.5 mmol). A solution of 4-amino-1H-imidazol-2-carboxylic acid (0.63 g, 5 mmol) and N-diisopropylethylamine in DMF (5 ml). The reaction mixture was stirred at ambient temperature overnight, the chloroform was evaporated and 1M aqueous sodium hydroxide was added to adjust the pH of the solution to 11.5. After 1 hour at ambient temperature, the solution was acidified to pH 5 with acetic acid. The pure compound was obtained after subjecting to HP20SS resin chromatography with a gradient of acetonitrile (0-50%) in water containing 1% acetic acid and freeze drying (410 mg, 18%).
NMR: δ 1.82 (m, 1H); 2.70 (m, 1H); 3.20-3.44 (m, 1H); 3.99 (m, 1H); 4.42 (m, 1H); 5.02-5.28 (m, 2H); 7.37 (s, 1H); 7.76 (d, 1H); 7.66 (d, 1H); 7.95 (d, 1H); 8.27 (d, 1H).

### 4-Nitrobenzyl (1R,5S,6S,8R,2'S,4'S)-2-(1-(4-nitrobenzyloxycarbonyl)-2-(2-carboxy-1H-imidazol-4-ylcarbamoyl)pyrrolidin-4-ylthio)-6-(1-hydroxyethyl)-1-methylcarbapenem-3-carboxylate, N-diisopropylethylamine salt.

To a solution of 4-nitrobenzyl (1R,5S,6S,8R)-6-(1-hydroxyethyl)-1-methyl-2-diphenylphosphoryloxycarbapenem-3-carboxylate (600 mg, 1 mmol) in DMF (10 ml) was added sequentially, at ambient temperature, N-diisopropylethylamine (0.5 ml, 2.9 mmol) and (2S,4S)-1-(4-nitrobenzyloxycarbonyl)-2-(2-carboxy-1H-imidazol-4-ylcarbamoyl)pyrrolidin-4-ylthio (400 mg, 0.9 mmol). The reaction mixture was stirred at ambient temperature for 2 hours and purified by subjecting to chromatography on HP20SS resin with a gradient of acetonitrile (0-45%) in water. Partial evaporation of the solvents and freeze drying gave the title compound (300 mg, 38%).
NMR: δ 1.17 (d, 3H); 1.18 (d, 3H); 1.24 (m, 9H); 1.84 (m, 1H); 2.78 (m, 1H); 3.13 (q, 2H); 3.25-3.42 (m, 2H); 3.56-3.67 (m, 3H); 3.89-4.03 (m, 2H); 4.09-4.30 (m, 2H); 4.49 (td, 1H); 5.04-5.47 (m, 4H); 7.27 (d, 1H); 7.47 (d, 1H); 7.59 (d, 1H); 7.67 (d, 1H); 7.70 (d, 1H); 7.96 (d, 1H); 8.18-8.26 (m, 3H).

### Example 5

### (1R,5S,6S,8R,2'S,4'S)-2-(2-(2-Carboxy-4-pyrrolylcarbamoyl)pyrrolidin-4-ylthio)-6-(1-hydroxyethyl)-1-methylcarbapenem-3-carboxylic acid.

A solution of 4-nitrobenzyl (1R,5S,6S,8R,2'S,4'S)-2-(1-(4-nitrobenzyloxycarbonyl)-2-(2-carboxy-4-pyrrolylcarbamoyl)pyrrolidin-4-ylthio)-6-(1-hydroxyethyl)-1-methylcarbapenem-3-carboxylate (250 mg, 0.32 mmol) in ethyl acetate (10 ml) and water (10 ml) with K₂CO₃ (0.075 g, 0.75 mmol) was hydrogenated at atmospheric pressure with Pd/carbon (10%, 0.2 g), the reaction being followed by HPLC. The mixture was filtered, the filtrate concentrated, and purified by preparative HPLC (C₁₈ Nucleosil), eluant water. The required fractions were collected, concentrated and freeze dried to give the title product (84 mg, 48%).
NMR: δ 1.15 (2d, 6H); 1.75 (m, 1H); 2.65 (m, 1H); 2.82 (dd, 1H); 3.2 (dd, 1H); 3.3-3.5 (m, 2H); 3.65 (m, 1H); 3.85-4.02 (m, 2H); 4.15 (dd, 1H); 6.78 (s, 1H); 7.24 (s, 1H).
The starting material was prepared as follows:

### 4-Amino-2-pyrrolecarboxylic acid.

4-Nitro-2-pyrrolecarboxylic acid (0.5 g, 3.2 mmol) in ethanol (10 ml) and water (20 ml) was hydrogenated over Pd/carbon (10%, 100 mg) at atmospheric pressure. After two hours the reaction was over, the catalyst was filtered off over diatomaceous earth, and the ethanol evaporated; the resulting aqueous solution was freeze dried to give the title compound (0.35 g, 88%).
NMR (DMSO-d₆) δ 6.13 (s, 1H); 6.28 (s, 1H).

### (2S,4S)-1-(4-Nitrobenzyloxycarbonyl)-2-(2-carboxy-4-pyrrolylcarbamoyl)pyrrolidin-4-ylthioacetate.

(2S,4S)-1-(4-Nitrobenzyloxycarbonyl)-4-acetylthio-2-carboxy-pyrrolidine (1 g, 2.72 mmol) was solubilized, at ambient temperature, in CH₂Cl₂ (5 ml) in the presence of thionyl chloride (1 ml, 13.7 mmol) and DMF (15 µl). The mixture was stirred for 4 hours, at ambient temperature, the solvent evaporated, the residual oil dissolved in CH₂Cl₂/toluene 1/1 (10 ml) and evaporated and dried under high vacuum for 1 hour. The residue was then solubilized in CH₂Cl₂ (10 ml). This solution was added to a cold solution (0°C) of 4-amino-2-pyrrole-carboxylic acid (0.37 g, 2.7 mmol), diisopropylethylamine (1.4 ml, 11.03 mmol) and trimethylsilyl chloride (1.9 ml, 10.93 mmol) in CH₂Cl₂ (anhydrous, 40 ml), and the mixture was stirred at ambient temperature for 2 hours. The solvent was evaporated, the residue dissolved in 2M hydrochloric acid, extracted with ethyl acetate, the organic phase washed with water (three times), dried and filtered to give a solid which was purified by subjecting to HP20SS chromatography (230 ml), using CH₃CN/H₂O/ACOH (50/50/1) as eluant. The required fractions were collected and freeze dried to give the title product (0.6 g, 47%).
NMR (DMSO-d₆) δ 1.88 (m, 1H); 2.32 (s, 3H); 2.7 (m, 1H); 3.35 (m, 1H); 3.88-4.12 (m, 2H); 4.38 (m, 1H); 5.0-5.32 (m, 2H); 6.65 (s, 1H); 7.18 (m, 1H); 7.49 (d, 1H); 7.66 (d, 1H); 7.96 (d, 1H); 8.24 (d, 1H).

### 4-Nitrobenzyl(1R,5R,6S,8R,2'S,4'S)-2-(1-(4-nitrobenzyloxycarbonyl)-2-(2-carboxy-4-pyrrolylcarbamoyl)pyrrolidin-4-ylthio)-6-(1-hydroxyethyl)-1-methylcarbapenem-3-carboxylate.

(2S,4S)-1-(4-Nitrobenzyloxycarbonyl)-2-(2-carboxy-4-pyrrolylcarbamoyl)pyrrolidin-4-ylthioacetate (0.33 g, 0.69 mmol) in methanol (20 ml) was treated with (1.15 ml, 1.15 mmol) at ambient temperature. After 1 hour the mixture was evaporated, acidified with 2M hydrochloric acid, saturated with ethyl acetate, washed with water (three times), dried over MgSO₄, filtered and evaporated to give a yellow foam. The crude thiol thus obtained was solubilized in CH₃CN (5 ml) and reacted with 4-nitrobenzyl (1R,5R,6S,8R)-6-(1-hydroxyethyl-1-methyl-2-diphenylphosphoryloxycarbapenem-3-carboxylate (0.4 g, 0.673 mmol) in CH₃CN (5 ml), diisopropylethylamine (0.265 ml, 1.63 mmol), tri-n-butyl-phosphine (0.035 ml, 0.14 mmol) and water 3 µl, 0.16 mmol) for 1 hour at ambient temperature, overnight at 4°C. The reaction product was purified by subjecting to HP20SS chromatography (100 ml) using CH₃CH/H₂O (40/60) with a gradient of CH₃CN as the eluant, to give the title compound after concentration and freeze drying of the required fractions. (0.26 g, 50%).
NMR: δ 1.81 (2d, 6H); 1.88 (m, 1H); 3.4 (m, 1H); 3.85-4.07 (m, 2H); 4.21 (m, 1H); 4.5-4.6 (m, 2H); 4.6-5.12 (m, 2H); 5.7 (d, 1H); 5.8-5.98 (m, 3H); 6.05 (d, 1H); 7.31 (s, 1H); 7.85 (m, 1H); 8.12 (d, 1H); 8.25-8.39 (m, 3H); 8.58 (d, 1H); 8.78-8.91 (m, 3H).

## Claims

1. A compound of the formula (I): 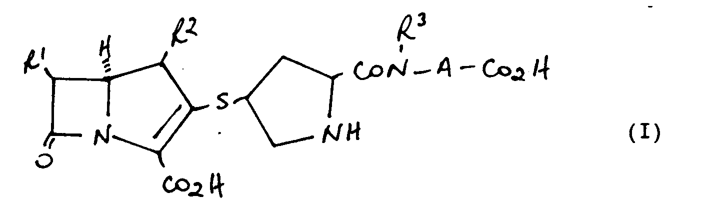 wherein:
R¹ is 1-hydroxyethyl, 1-fluoroethyl or hydroxymethyl;
R² is hydrogen or C₁₋₄alkyl;
R³ is hydrogen or C₁₋₄alkyl;
A is a 5-membered heteroaryl ring containing one nitrogen atom and up to two additional heteroatoms selected from nitrogen, oxygen and sulphur; and is bonded to the nitrogen of the linking carbamoyl group by a carbon atom in the ring, is substituted with the carboxy group on a carbon atom in the ring and is optionally further substituted on a carbon atom in the ring by halo, cyano, C₁₋₄alkyl, nitro, hydroxy, carboxy, C₁₋₄alkoxy, trifluoromethyl, C₁₋₄alkoxycarbonyl, amino, C₁₋₄alkylamino, di-C₁₋₄alkylamino, C₁₋₄alkylS(O)ₙ- (wherein n is 0-2), C₁₋₄alkanoylamino, C₁₋₄alkanoyl(N-C₁₋₄alkyl)amino, carbamoyl, C₁₋₄alkylcarbamoyl or di-C₁₋₄alkylcarbamoyl; and
in any ring -NH-, H is optionally replaced by C₁₋₄alkyl; or a pharmaceutically acceptable salt or in vivo hydrolysable ester thereof;

2. A compound according to claim 1 wherein R² is methyl.

3. A compound according to claim 1 wherein R¹ is 1-hydroxyethyl.

4. A compound according to either claim 1 or claim 2 of the formula (IV): 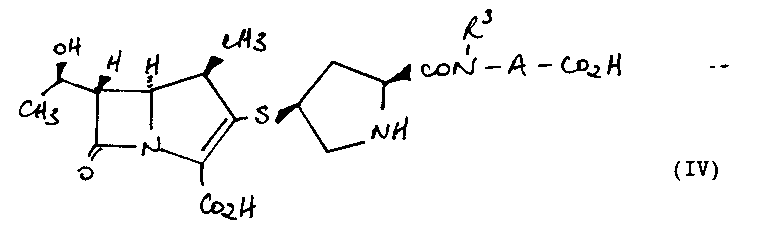 wherein A, R³, and optional substituents on A are as defined in claim 1.

5. A compound according to claim 4 wherein optional substituents on A are selected from halo, cyano, C₁₋₄alkyl, nitro, hydroxy, carboxy, C₁₋₄alkoxy, carbamoyl, amino and trifluoromethyl.

6. A compound according to claim 1 which is
(1R,5S,6S,8R,2'S,4'S)-2-(2-(5-carboxythiazol-2-ylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(2-carboxy-1H-imidazol-4-ylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(2-carboxypyrrol-4-ylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(4-carboxythiazol-2-ylcarbamoyl)pyrrolidin-4-ylthio)-6-(1-hydroxyethyl)-1-methylcarbapenem-3-carboxylic acid; and
(1R,5S,6S,8R,2'S,4'S)-2-(2-(5-carboxy-1,3,4-thiadiazol-2-ylcarbamoyl)-pyrrolidin-4-ylthio)-6-(1-hydroxyethyl)-1-methylcarbapenem-3 carboxylic acid;
and pharmaceutically acceptable salt thereof.

7. A pharmaceutical composition which comprises a compound according to any one of claims 1 to 6 and a pharmaceutically acceptable carrier.

8. A process for preparing a compound according to claim 1 which comprises deprotecting a compound of the formula (V): 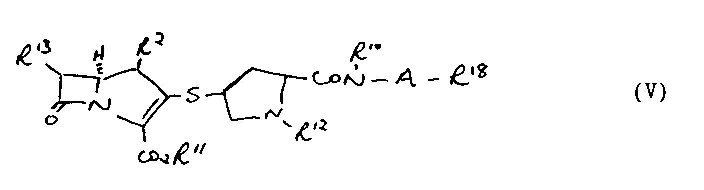 wherein R² is as defined in claim 1; R¹⁰ is a group R³ (as defined in claim 1) or an amino protecting group; R¹³ is a group R¹ (as defined in claim 1), protected hydroxymethyl or 1-(protected hydroxy)ethyl; R¹¹ is hydrogen or a carboxy protecting group; R¹² is hydrogen or an amino protecting group; A is as defined in claim 1;
R¹⁸ is a carboxy group or a protected carboxy group and wherein any optional substituent on A is as defined in claim 1 and is optionally protected; and wherein at least one protecting group is present; and thereinafter if necessary;
(i) forming a pharmaceutically acceptable salt,
(ii) esterifying to form an in vivo hydrolysable ester.

9. A process for preparing a compound according to claim 1 or a compound of the formula (V) as defined in claim 8 which comprises:
a) reacting compounds of the formulae (VI) and (VII): 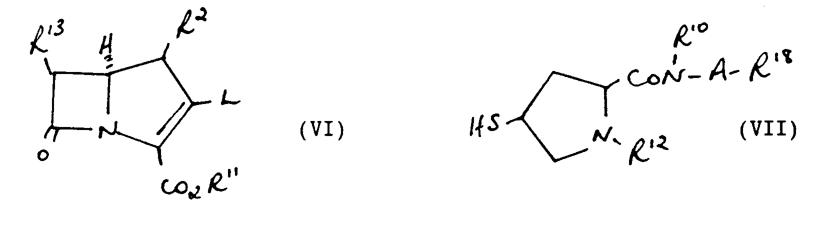 wherein A, R², R¹⁰ R¹¹, R¹², R¹³ and R¹⁸ are as defined in claim 8, optional substituents on A are as defined in claim 8 and L is a leaving group, or
b) cyclising a compound of the formula (VIII): 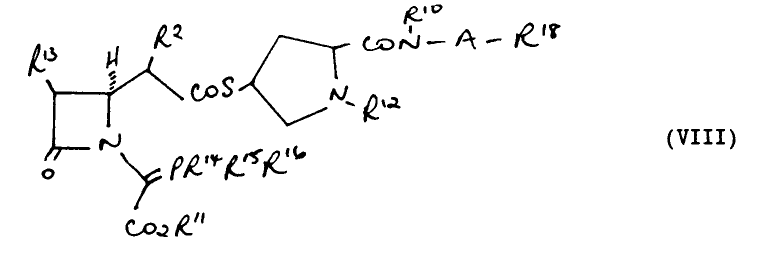 A, R² R¹⁰, R¹¹, R¹², R¹³ and R¹⁸ are as defined in claim 8, optional substituents on A are as defined in claim 8 and R¹⁴, R¹⁵ and R¹⁶ are independently selected from C₁₋₆alkoxy, aryloxy, di-C₁₋₆alkylamino and diarylamino or any two of R¹⁴-R¹⁶ represent o-phenylenedioxy or one of R¹⁴-R¹⁶ is C₁₋₄alkyl, allyl, benzyl or phenyl and the other two values are independently selected from C₁₋₄alkyl, trifluoromethyl or phenyl, wherein any phenyl group is optionally substituted with C₁₋₃alkyl or C₁₋₃alkoxy; and wherein any functional group is optionally protected and thereinafter if necessary:
(i) removing any protecting groups;
(ii) forming a pharmaceutically acceptable salt;
(iii) esterifying to form an in vivo hydrolysable ester.

10. A compound of the formula (V) as defined in claim 8, of the formula (VII) or (VIII) as defined in claim 9, or of the formula (IX), (XII) or (XIV): 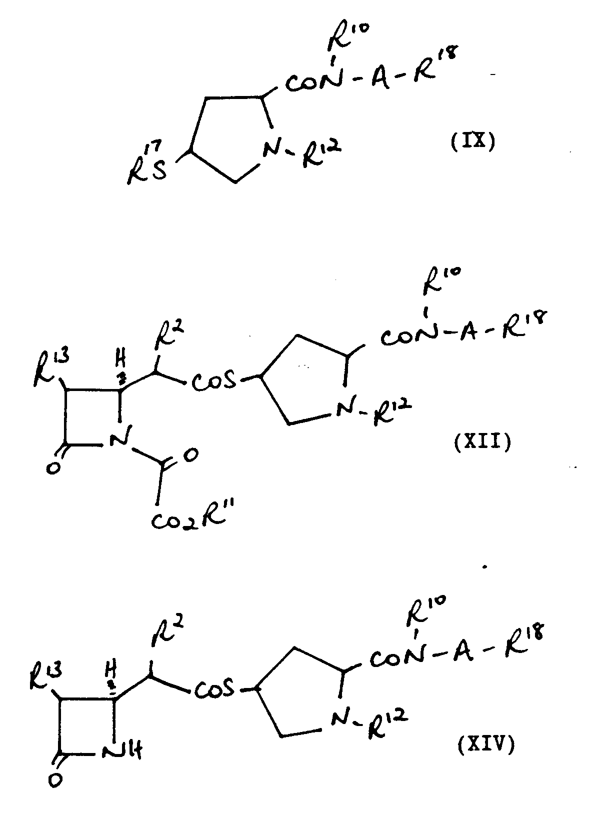 wherein A, R², R¹⁰-R¹³ and R¹⁸ are as defined in claim 8 and R¹⁷ is a protecting group.

## Patentansprüche

1. Verbindung der Formel (I): 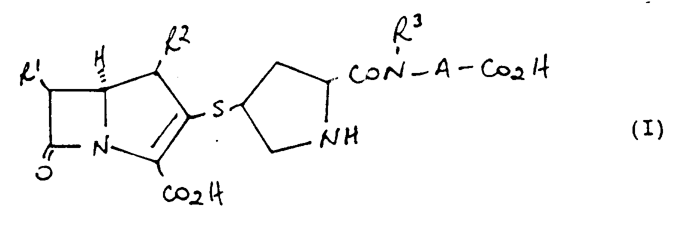 wobei:
R¹ eine 1-Hydroxyethyl-, 1-Fluorethyl- oder Hydroxymethylgruppe ist;
R² ein Wasserstoffatom oder ein C₁₋₄-Alkylrest ist;
R³ ein Wasserstoffatom oder ein C₁₋₄-Alkylrest ist;
A ein 5-gliedriger Heteroarylring mit einem Stickstoffatom und bis zu zwei zusätzlichen Heteroatomen, ausgewählt aus Stickstoff, Sauerstoff und Schwefel, ist; und durch ein Kohlenstoffatom im Ring an das Stickstoffatom der verbindenden Carbamoylgruppe gebunden ist, an einem Kohlenstoffatom im Ring mit der Carboxygruppe substituiert ist und gegebenenfalls an einem Kohlenstoffatom im Ring durch ein Halogenatom, eine Cyanogruppe, einen C₁₋₄-Alkyrest, eine Nitro-, Hydroxy-, Carboxygruppe, einen C₁₋₄-Alkoxyrest, eine Trifluormethylgruppe, einen C₁₋₄-Alkoxycarbonylrest, eine Aminogruppe, einen C₁₋₄-Alkylamino-, Di-C₁₋₄-alkylamino-, C₁₋₄-Alkyl-S(O)ₙ- (wobei n 0-2 ist), C₁₋₄-Alkanoylamino-, C₁₋₄-Alkanoyl(*N*-C₁₋₄-alkyl)aminorest, eine Carbamoylgruppe, einen C₁₋₄-Alkylcarbamoyl- oder Di-C₁₋₄-Alkylcarbamoylrest weiter substituiert ist; und
in jedem Ring -NH-, H gegebenenfalls durch C₁₋₄-Alkylreste ersetzt ist;
oder ein pharmazeutisch verträgliches Salz oder ein in vivo hydrolysierbarer Ester davon.

2. Verbindung nach Anspruch 1, wobei R² eine Methylgruppe ist.

3. Verbindung nach Anspruch 1, wobei R¹ eine 1-Hydroxyethylgruppe ist.

4. Verbindung nach Anspruch 1 oder Anspruch 2 der Formel (IV): 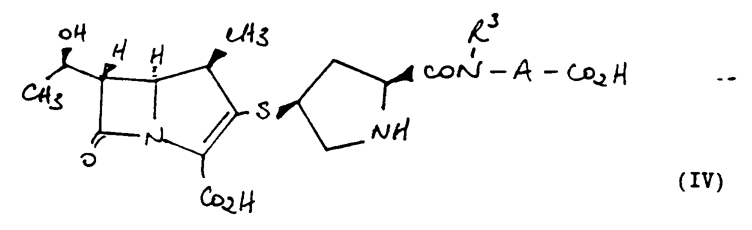 wobei A, R³ und mögliche Substituenten an A die in Anspruch 1 angegebene Bedeutung haben.

5. Verbindung nach Anspruch 4 wobei mögliche Substituenten an A aus Halogenatomen, Cyanogruppen, C₁₋₄-Alkylresten, Nitro-, Hydroxy-, Carboxygruppen, C₁₋₄-Alkoxyresten, Carbamoyl-, Amino- und Trifluormethylgruppen ausgewählt sind.

6. Verbindung nach Anspruch 1, die
(1R,5S,6S,8R,2'S,4'S)-2-(2-(5-Carboxythiazol-2-ylcarbamoyl)pyrrolidin-4-ylthio)-6-(1-hydroxyethyl)-1-methylcarbapenem-3-carbonsäure:
(1R,5S,6S,8R,2'S,4'S)-2-(2-(2-Carboxy-1H-imidazol-4-ylcarbamoyl)pyrrolidin-4-ylthio)-6-(1-hydroxyethyl)-1-methylcarbapenem-3-carbonsäure;
(1R,5S,6S,8R,2'S,4'S)-2-(2-2-Carboxypyrrol-4-ylcarbamoyl)pyrrolidin-4-ylthio)-6-(1-hydroxyethyl)-1-methylcarbapenem-3-carbonsäure;
(1R,5S,6S,8R,2'S,4'S)-2-(2-(4-Carboxythiazol-2-ylcarbamoyl)pyrrolidin-4-ylthio)-6-(1-hydroxyethyl)-1-methylcarbapenem-3-carbonsäure; und
(1R,5S,6S,8R,2'S,4'S)-2-(2-(5-Carboxy-1,3,4-thiadiazol-2-ylcarbamoyl)pyrrolidin-4-ylthio)-6-(1-hydroxyethyl)-1-methylcarbapenem-3-carbonsäure ist;
und pharmazeutisch verträgliche Salze davon.

7. Arzneimittel, das eine Verbindung nach einem der Ansprüche 1 bis 6 und einen pharmazeutisch verträglichen Träger umfaßt.

8. Verfahren zur Herstellung einer Verbindung nach Anspruch 1, das die Schutzgruppenabspaltung aus einer Verbindung der Formel (V) umfaßt: 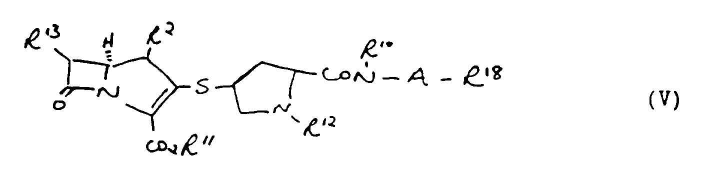 wobei R² die in Anspruch 1 angegebene Bedeutung hat; R¹⁰ ein Rest R³ (der die in Anspruch 1 angegebene Bedeutung hat) oder eine Aminschutzgruppe ist; R¹³ ein Rest R¹ (der die in Anspruch 1 angegebene Bedeutung hat), eine geschützte Hydroxymethylgruppe oder 1-(geschützte Hydroxy)ethylgruppe ist; R¹¹ ein Wasserstoffatom oder eine Carboxyschutzgruppe ist; R¹² ein Wasserstoffatom oder eine Aminschutzgruppe ist; A die in Anspruch 1 angegebene Bedeutung hat; R¹⁸ eine Carboxygruppe oder ein geschützter Carboxyrest ist, und wobei jeder mögliche Substituent an A die in Anspruch 1 angegebene Bedeutung hat und gegebenenfalls geschützt ist; und wobei mindestens eine Schutzgruppe vorhanden ist; und danach falls nötig;
(i) Erzeugung eines pharmazeutisch verträglichen Salzes,
(ii) Veresterung zu einem in vivo hydrolysierbaren Ester.

9. Verfahren zur Herstellung einer Verbindung nach Anspruch 1 oder einer Verbindung der Formel (V), die die in Anspruch 8 angegebene Bedeutung hat, das umfaßt:
a) die Umsetzung von Verbindungen der Formeln (VI) und (VII): 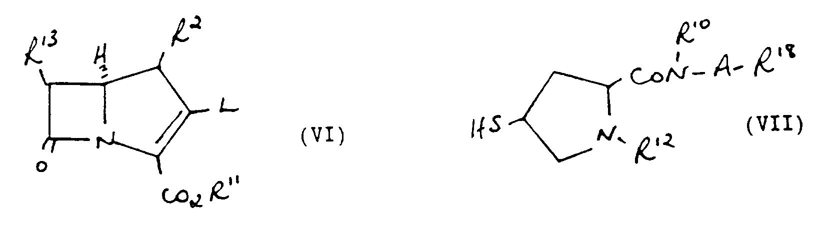 wobei A, R², R^{10,} R¹¹, R¹², R¹³ und R¹⁸ die in Anspruch 8 angegebene Bedeutung haben, mögliche Substituenten an A die in Anspruch 8 angegebene Bedeutung haben und L eine Abgangsgruppe darstellt, oder
b) die Cyclisierung einer Verbindung der Formel (VIII): 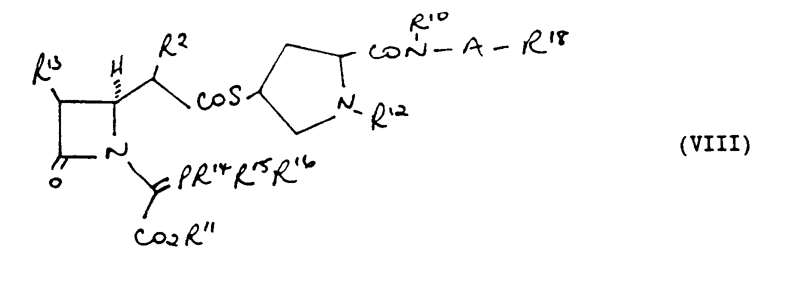 wobei A, R², R¹⁰, R¹¹, R¹², R¹³ und R¹⁸ die in Anspruch 8 angegebene Bedeutung haben, mögliche Substituenten an A die in Anspruch 8 angegebene Bedeutung haben und R¹⁴, R¹⁵ und R¹⁶ unabhängig aus C₁₋₆-Alkoxy-, Aryloxy-, Di-C₁₋₆-alkylamino- und Diarylaminoresten ausgewählt sind oder jeweils zwei der Reste R¹⁴-R¹⁶ eine *o*-Phenylendioxygruppe darstellen oder einer der Reste R¹⁴-R¹⁶ ein C₁₋₄-Alkylrest oder eine Allyl-, Benzyl-, oder Phenylgruppe ist und die anderen beiden Werte unabhängig aus C₁₋₄-Alkylresten, Trifluormethyl- oder Phenylgruppen ausgewählt sind, wobei jede Phenylgruppe gegebenenfalls mit C₁₋₃-Alkyl- oder C₁₋₃-Alkoxyresten substituiert ist; und wobei jede funktionelle Gruppe gegebenenfalls geschützt ist und danach, falls nötig:
(i) Entfernen der Schutzgruppen;
(ii) Erzeugung eines pharmazeutisch verträgtichen Salzes;
(iii) Veresterung zu einem in vivo hydrolysierbaren Ester.

10. Verbindung der Formel (V), die die in Anspruch 8 angegebene Bedeutung hat, der Formel (VII) oder (VIII), die die in Anspruch 9 angegebene Bedeutung haben, oder der Formel (IX), (XII) oder (XIV): 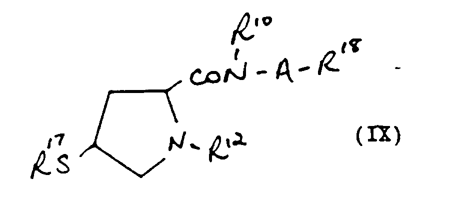 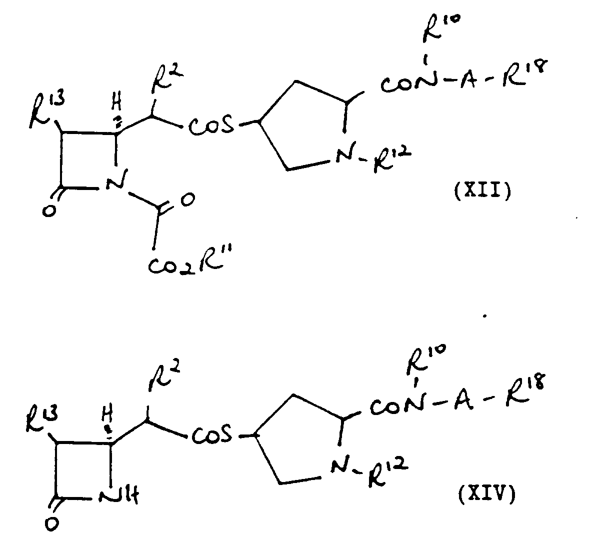 wobei A, R², R¹⁰-R¹³ und R¹⁸ die in Anspruch 8 angegebene Bedeutung haben und R¹⁷ eine Schutzgruppe ist.

## Revendications

1. Composé de Formule (I): 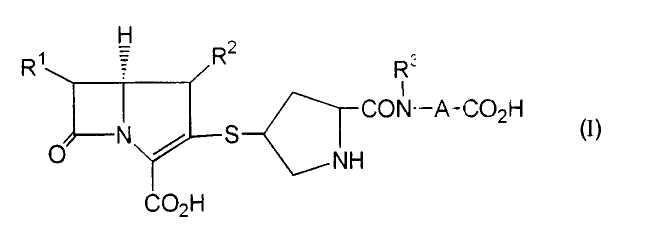 dans laquelle:
R¹ est un groupe 1-hydroxyethyle, 1-fluoroethyle ou hydroxyméthyle;
R² est l'hydrogène ou un groupe alkyle en C1-4;
R³ est l'hydrogène ou un groupe alkyle en C1-4;
A est un cycle hétéroaryle a 5 membres contenant un atome d'azote et jusqu'a deux hétéroatomes supplémentaires choisis parmi l'azote, l'oxygène et le soufre; et est lié à l'azole du groupe carbamoyle liant par un atome de carbone dans le cycle, est substitue par un groupe carboxy sur l'atome de carbone dans le cycle, et est éventuellement encore substitué sur l'atome de carbone dans le cycle, par un groupe halo, cyano, alkyle en C1-4, nitro, hydroxy, carboxy, alcoxy en C1-4, trifluorométhyle, alcoxycarbonyle en C1-4, amino, alkylamino en C1-4, di-alkylamino en C1-4, alkyl (en C1-4)S(O)ₙ- (dans lequel n vaut de 0 à 2), alcanoylamino en C1-4, alcanoyl (en C1-4) amino(N-alkyl en C1-4), alkylcarbamoyle en C1-4 ou di-alkylcarbamoyle en C1-4; et
dans un cycle quelconque -NH-, H est éventuellement remplacé par un groupe alkyle en C1-4;
ou un sel acceptable pharmaceutiquement ou un esier hydrolysable in vivo de celui-ci.

2. Composé selon la revendication 1, caractérisé en ce que R² est un groupe méthyle.

3. Compose selon la revendication 1, caractérisé en ce que R¹ est un groupe 1-hydroxyéthyle.

4. Composé selon la revendication1 ou la revendication 2, de Formule (IV): 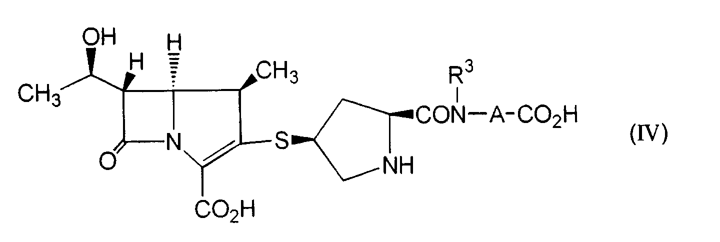 dans laquelle A, R³ et les substituants éventuels sur A sont tels que définis dans la revendication 1.

5. Composé selon la revendication 4, caractérisé en ce que les substituants éventuels sur A sont choisis parmi un groupe halo, cyano, alkyle en C1-4, niiro, hydroxy, carboxy, alcoxy en C1-4, carbamoyle, amino et trifluorométhyle.

6. Composé selon la revendication 1, caractérisé en ce qu'il est
l'acide (1R, 5S, 6S, 8R, 2'S, 4'S)-2-(2-(5-carboxythiazol-2-ylcarbamoyl) pyrrolidin-4-ylthio)-6-(1-hydroxyéthy)-1-méthylcarbapénèm-3-carboxylique;
l'acide (1R, 5S, 6S, 8R, 2'S, 4'S)-2-(2-(2-carboxy-1H-imidazol-4-ylcarbamoyl) pyrroidin-4-ylthio)-6-(1-hydroxyéthyl)-1-méthylcarbapénèm-3-carboxylique;
l'acide (1R, 5S, 6S, 8R, 2'S, 4'S)-2-(2-(2-carboxypyrrol-4-ylcarbamoyl) pyrrolidin-4-ylthio)-6-(1-hydroxyéthyl)-1-méthylcarbapénèm-3 carboxylique;
l'acide (1R, 5S, 6S, 8R, 2'S, 4'S)-2-(2-(4-carboxythiazol-2-ylcarbamoyl) pyrrolidin-4-ylthio)-6-(1-hydroxyéthyl)-1-méthylcarbapénèm-3 carboxylique; et
l'acide (1R, 5S, 6S, 8R, 2'S, 4'S)-2-(2-(5-carboxy-1,3,4-thiadiazol-2-ylcarbamoyl)pyrrolidin-4-ylthio)-6-(1-hydroxyéthyl)-1-méthylcarbapénèm-3 carboxylique;
et un sel acceptable pharmaceutiquement de celui-ci.

7. Composition pharmaceutique qui comprend un composé selon l'une quelconque des revendications 1 à 6 et un support acceptable pharmaceutiquement.

8. Procédé de préparaiion d'un composé selon la revendication 1 qui comprend la déprotection d'un composé Formule (V): 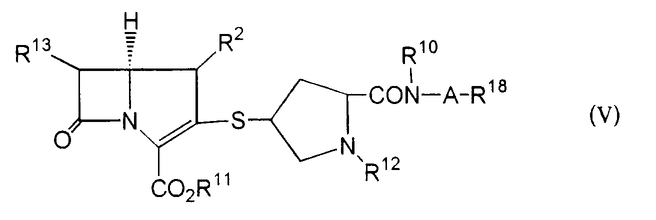 dans laquelle R² est tel que défini dans la revendication 1; R¹⁰ est un groupe R³ (tel que défini dans la revendication 1) ou un groupe protecteur de groupe amino; R¹³ est un groupe R¹ (tel que défini dans la revendication 1), hydroxyméthyle protégé ou 1-(hydroxy protégé)éthyle; R¹¹ est l'hydrogène ou un groupe protecteur de groupe carboxy; R¹² est l'hydrogène ou un groupe protecteur de groupe amino; A est tel que défini dans la revendication 1;
R¹⁸ est un groupe carboxy ou un groupe carboxy protégé et dans laquelle tout substituant éventuel sur A est tel que défini dans la revendication 1 et est éventuellement protégé; et dans laquelle au moins un groupe protecteur est présent; et ensuite si nécessaire;
(i) la formation d'un sel acceptable pharmaceutiquement,
(ii) l'estérification pour former un ester hydrolysable in vivo.

9. Procédé de préparation d'un composé selon la revendication 1 ou un composé de Formule (V) telles que définie dans la revendication 1 qui comprend:
a) la réaction de composés de Formule (VI) et (VII): 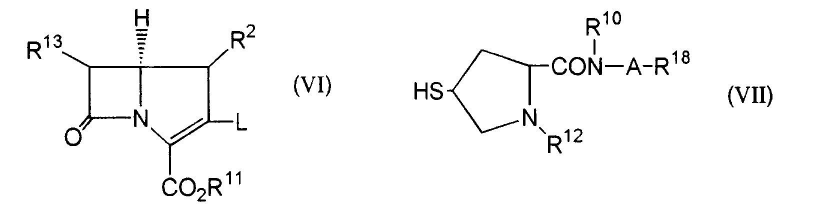 dans lequelles A, R², R¹⁰, R¹¹, R¹², R¹³ et R¹⁸ sont tels que définisdans la revendication 8, les substituants éventuels sur A étant tels que définis dans la revendication 8 et L étant un groupe se séparant, ou
b) la cyclisation d'un composé de Formule (VIII): 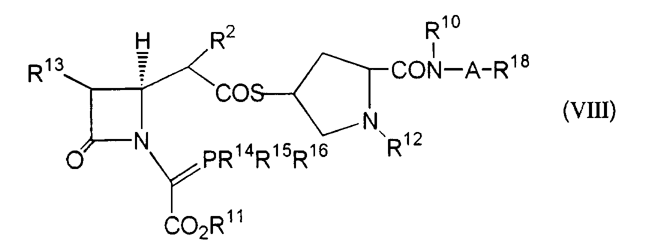 dans laquelle A, R², R¹⁰, R¹¹, , R1¹², R¹³ et R¹⁸ sont tels que définis dans la revendication 8; les substituants éventuels sur A étant tels que définis dans la revendication 8 et R¹⁴, R¹⁵ et R¹⁶ sont indépendamment choisis parmi un groupe alcoxy en C1-6, aryloxy, dialkylamino en C1-6 et diarylamino ou l'un quelconque de deux R¹⁴ -R¹⁶ représente un groupe o-
phénylènedioxy ou l'un quelconque des R¹⁴ -R¹⁶ est un groupe alkyle en C1-4, allyle, benzyle ou phényle et les deux autres valeurs sont indépendamment choises parmi un groupe alkyle en C1-4, trifluorométhyle ou phényle, l'un quelconque des groupes phényle étant éventuellement substitué par un groupe alkyle en C1-3 ou alcoxy en C1-3; et dans laquelle l'un quelconque des groupes fonctionnels étant éventuellement protégé et ensuite, si nécessaire:
(i) l'enlèvement de l'un quelconque des groupes protecteurs;
(ii) la formation d'un sel acceptable pharmaceutiquement;
(iii) l'estérification pour former un ester hydrolysable in vivo.

10. Composé de Formule (V) telle que définie dans la revendication 8, ou de formule (VII) ou (VIII) telles que définie dans la revendication 9, ou de Formule (IX), (XII) ou (XIV): 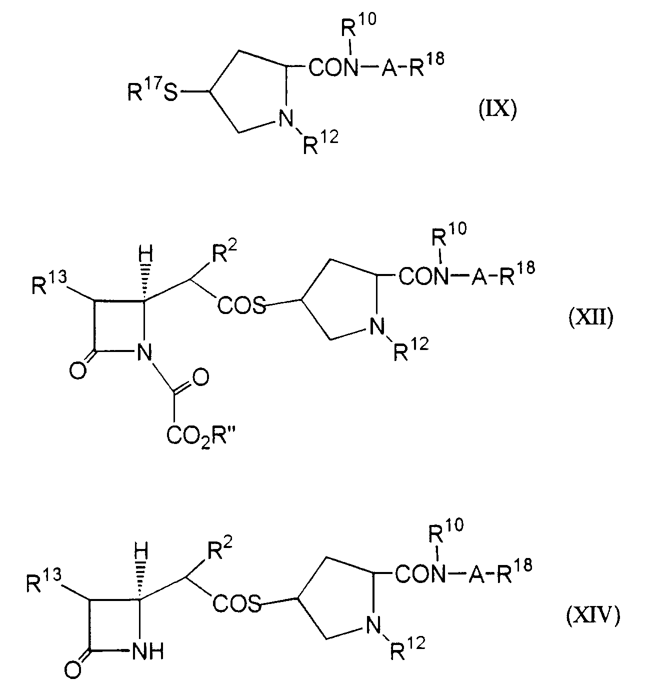 dans lesquelles A,R², R¹⁰-R¹³ et R¹⁸ sont tels que définis dans la revendication 8 et R¹⁷ est un groupe protecteur.
